(19) Europäisches Patentamt
European Patent Office
Office européen des brevets

(11) EP 0 554 829 B1

(12) EUROPEAN PATENT SPECIFICATION

(45) Date of publication and mention of the grant of the patent:
**15.05.2002 Bulletin 2002/20**

(51) Int Cl.[7]: **C07D 231/12**, C07D 231/16, C07D 405/04, C07D 231/14, A61K 31/415

(21) Application number: **93101569.7**

(22) Date of filing: **02.02.1993**

(54) **Pyrazole derivatives with antiinflammatory, analgesic and antithrombolic activity**

Pyrazolderivate mit entzündunghemmender, analgetischer und antithrombotischer Wirkung

Dérivés du pyrazole à activité anti-inflammatoire, analgésique et antithrombotique

(84) Designated Contracting States:
**AT BE CH DE DK ES FR GB GR IE IT LI LU NL PT SE**

(30) Priority: **05.02.1992 GB 9202442**
**28.09.1992 GB 9220427**

(43) Date of publication of application:
**11.08.1993 Bulletin 1993/32**

(73) Proprietor: **FUJISAWA PHARMACEUTICAL CO., LTD.**
**Osaka-shi Osaka 541-8514 (JP)**

(72) Inventors:
- **Matsuo, Masaaki**
**Toyonaka-shi, Osaka 560 (JP)**
- **Tsuji, Kiyoshi**
**Kishiwada-shi, Osaka 596 (JP)**
- **Ogino, Takashi**
**Yamatokooriyama-shi, Nara 639-11 (JP)**
- **Konishi, Nobukiyo**
**Nagaokakyo-shi, Kyoto 617 (JP)**

(74) Representative:
**Gille Hrabal Struck Neidlein Prop Roos**
**Patentanwälte**
**Brucknerstrasse 20**
**40593 Düsseldorf (DE)**

(56) References cited:
**EP-A- 0 248 594**
**EP-A- 0 418 845**
**CA-A- 1 130 808**

Remarks:
The file contains technical information submitted after the application was filed and not included in this specification

EP 0 554 829 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention relates to new pyrazole derivatives and pharmaceutically acceptable salts thereof.

More particularly, it relates to new pyrazole derivatives and pharmaceutically acceptable salts thereof, which have antiinflammatory, analgesic and antithrombotic activities, to processes for preparation thereof, to a pharmaceutical composition comprising the same, and to methods of using the same therapeutically in the treatment and/or prevention of inflammatory conditions, various pains, collagen diseases, autoimmune diseases, various immunity diseases and thrombosis in human beings or animals, and more particularly to methods for the treatment and/or prevention of inflammation and pain in joint and muscle [e.g. rheumatoid arthritis, rheumatoid spondylitis, osteoarthritis, gouty arthritis, etc.], inflammatory skin condition [e.g. sunburn, eczema, etc.], inflammatory eye condition [e.g. conjunctivitis, etc.], lung disorder in which inflammation is involved [e.g. asthma, bronchitis, pigeon fancier's disease, farmer's lung, etc.], condition of the gastrointestinal tract associated with inflammation [e.g. aphthous ulcer, Crohn's disease, atropic gastritis, gastritis varialoforme, ulcerative colitis, coeliac disease, regional ileitis, irritable bowel syndrome, etc.], gingivitis, inflammation, pain and tumescence after operation or injury, pyresis, pain and other conditions associated with inflammation, particularly those in which lipoxygenase and cyclooxygenase products are a factor, systemic lupus erythematosus, scleroderma, polymyositis, periarteritis nodosa, rheumatic fever, Sjögren's syndrome, Behcet disease, thyroiditis, type I diabetes, nephrotic syndrome, aplastic anemia, myasthenia gravis, uveitis contact dermatitis, psoriasis, Kawasaki disease, sarcoidosis, Hodgkin's disease, and the like. Additionally, the object compound is expected to be useful as therapeutical and/or preventive agents for cardiovascular or cerebrovascular diseases, the diseases caused by hyperglycemia and hyperlipemia.

One object of this invention is to provide new and useful pyrazole derivatives and pharmaceutically acceptable salts thereof,which possess antiinflammatory, analgesic and antithrombotic activities.

Another object of this invention is to provide processes for the preparation of said pyrazole derivatives and salts thereof.

A further object of this invention is to provide a pharmaceutical composition comprising, as an active ingredient, said pyrazole derivatives and pharmaceutically acceptable salts thereof.

Still further object of this invention is to provide a therapeutical method for the treatment and/or prevention of inflammatory conditions, various pains, and the other diseases mentioned above, using said pyrazole derivatives and pharmaceutically acceptable salts thereof.

Some pyrazole derivatives having antiinflammatory and analgesic activities have been known as described, for example, in Canadian Patent 1 130 808, and EP Patent publication Nos. 272 704, 293 220 and 418 845.

The object pyrazole derivatives of this invention are new and can be represented by the following general formula [I].

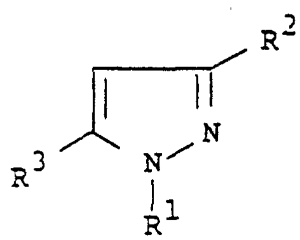

[I]

whereir

$R^1$ is phenyl which is substituted with substituent(s) selected from the group consisting of cyano, $(C_1\text{-}C_6)$alkylenedioxy, $(C_1\text{-}C_6)$alkanoyl and $(C_1\text{-}C_6)$alkoxy,

$R^2$ is halogen, halo $(C_1\text{-}C_6)$alkyl, or cyano, and

$R^3$ is phenyl substituted with $(C_1\text{-}C_6)$alkylthio, $(C_1\text{-}C_6)$alkylsulfinyl or $(C_1\text{-}C_6)$alkylsulfonyl,

provided, that when

$R^1$ is phenyl substituted with $(C_1\text{-}C_6)$alkoxy
then
$R^2$ is halogen or halo$(C_1\text{-}C_6)$alkyl

and pharmaceutically acceptable salts thereof.

The object compound [I] or its salt can be prepared by the following processes.

Process 1

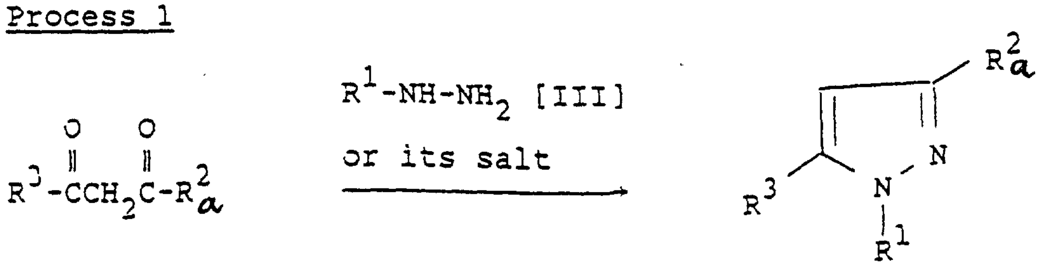


[II] or its salt

[Ia] or its salt

Process 2

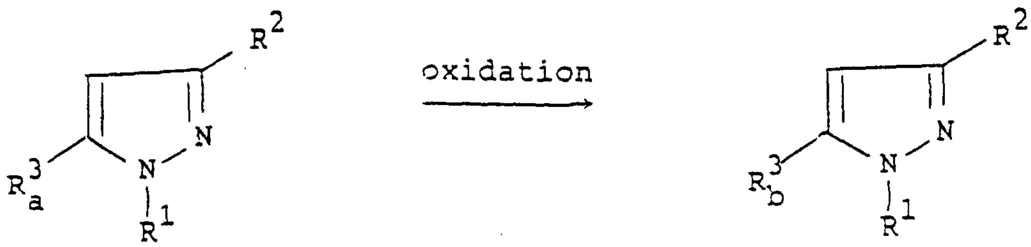


[Ib] or its salt

[Ic] or its salt

Process 3

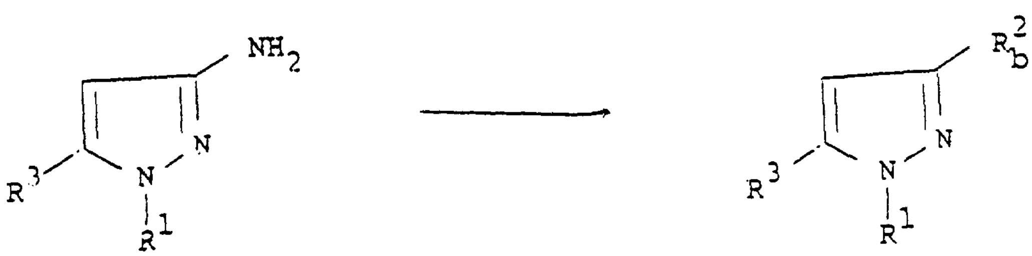

[IV] or its salt

[Id] or its salt

Process 4

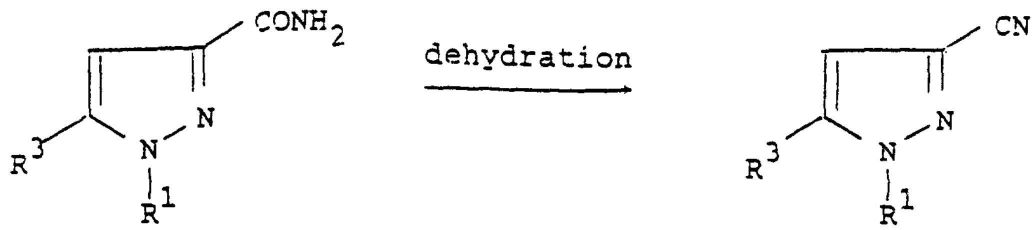


[Ik] or its salt

[Iℓ] or its salt

Process 5

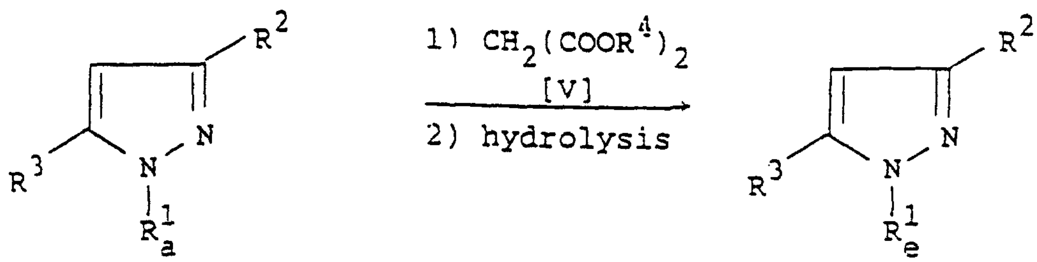


[Ie]
or its reactive derivative
at the carboxy group
or a salt thereof

[Im]

Process 6

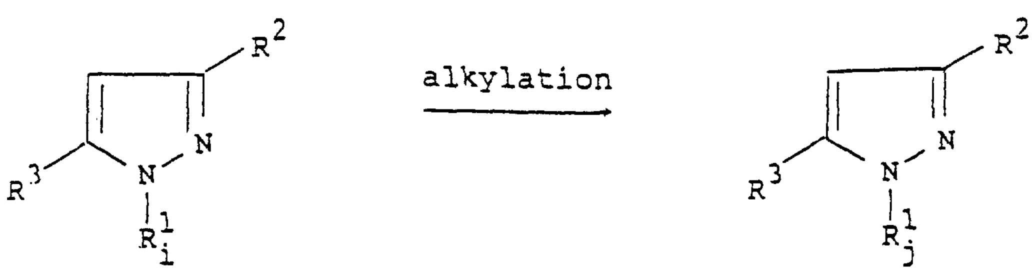


[Iq] or its salt

[Ir] or its salt

wherein

| | |
|---|---|
| $R^1$, $R^2$ and $R^3$ | are each as defined above, |
| $R^2_a$ | is halo($C_1$-$C_6$)alkyl or cyano, |
| $R^3_a$ | is phenyl substituted with ($C_1$-$C_6$)alkylthio, |
| $R^3_b$ | is phenyl substituted with ($C_1$-$C_6$)alkylsulfinyl or ($C_1$-$C_6$)alkylsulfonyl, |
| $R^2_b$ | is halogen, |
| $R^1_a$ | is phenyl substituted with carboxy, |
| $R^1_e$ | is phenyl substituted with acetyl, |
| $R^4$ | is ($C_1$-$C_6$)alkyl, |
| $R^1_i$ | is phenyl substituted with hydroxy, |
| $R^1_j$ | is phenyl substituted with ($C_1$-$C_6$)alkoxy. |

In the above and subsequent description of the present specification, suitable examples of the various definitions to be included within the scope of the invention are explained in detail in the following.

Suitable " ($C_1$-$C_6$)alkyl" and ($C_1$-$C_6$)alkyl moiety in the terms " ($C_1$-$C_6$) alkylthio", " ($C_1$-$C_6$)alkylsulfonyl", "($C_1$-$C_6$) alkylsulfinyl", "hydroxy ($C_1$-$C_6$)alkyl" and "halo ($C_1$-$C_6$)alkyl" may be a straight or branched one such as methyl, ethyl, propyl, isopropyl, butyl, isobutyl, tert-butyl, pentyl and hexyl, in which preferable one is methyl.

Suitable " ($C_1$-$C_6$)alkoxy" may be methoxy, ethoxy, propoxy, isopropoxy, butoxy, isobutoxy and tert-butoxy, in which preferable one is methoxy, ethoxy or isopropoxy.

Suitable "halogen" may be fluorine, chlorine, bromine and iodine, in which preferable one is bromine.

Suitable "halo ($C_1$-$C_6$)alkyl" may be chloromethyl, fluoromethyl, bromomethyl, difluoromethyl, dichloromethyl, trifluoromethyl, trichloromethyl and 2-fluoroethyl, in which preferable one is difluoromethyl or trifluoromethyl.

Suitable " ($C_1$-$C_6$)alkylenedioxy" may be a straight or branched one such as methylenedioxy, ethylenedioxy, trimethylenedioxy, dimethylmethylenedioxy and propylenedioxy.

The ($C_1$-$C_6$)alkanoyl may be formyl, acetyl, propionyl, butyryl, isobutyryl, valeryl, isovaleryl, pivaloyl and hexanoyl.

Suitable "($C_1$-$C_6$)alkylthio" may be methylthio, ethylthio and propylthio, in which preferable one is methylthio.

Suitable" ($C_1$-$C_6$)alkylsulfonyl " may be methylsulfonyl, ethylsulfonyl and propylsulfonyl, in which preferable one is methylsulfonyl.

Suitable "($C_1$-$C_6$)alkylsulfinyl" may be methylsulfinyl, ethylsulfinyl and propylsulfinyl, in which preferable one is methylsulfinyl.

Preferable compound [I] is one which has phenyl substituted with cyano, ($C_1$-$C_6$)alkanoyl or ($C_1$-$C_6$)alkoxy for $R^1$, halogen or halo' ($C_1$-$C_6$)alkyl for $R^2$ and phenyl substituted with ($C_1$-$C_6$)-alkylthio, ($C_1$-$C_6$)alkylsulfinyl or ($C_1$-$C_6$) alkylsulfonyl for $R^3$.

More preferable compound [I] is one which has phenyl substituted with methoxy or cyano for $R^1$, bromine, difluoromethyl or trifluoromethyl for $R^2$ and phenyl substituted with methylthio, methylsulfinyl or methylsulfonyl for $R^3$, or phenyl substituted with methoxy for $R^1$, bromine or difluoromethyl for $R^2$ and phenyl substituted with methylthio, methylsulfinyl or methylsulfonyl for $R^3$.

Most preferable compound [I] is one which has 4-methoxyphenyl for $R^1$, bromine or difluoromethyl for $R^2$, or 4-cyanophenyl for $R^1$, trifluoromethyl for $R^2$, and 4-(methylthio)phenyl, 4-(methylsulfinyl)phenyl or 4-(methylsulfonyl) phenyl for $R^3$.

Suitable pharmaceutically acceptable salts of the object compound [I] are conventional non-toxic salts and include a metal salt such as an alkali metal salt [e.g. sodium salt, potassium salt, etc.], and an alkaline earth metal salt [e.g. calcium salt, magnesium salt, etc.].

The processes for preparing the object compound [I] are explained in detail in the following.

Process 1

The compound [Ia] or its salt can be prepared by reacting a compound [II] or its salt with a compound [III] or its salt.

Suitable salts of the compounds [Ia] and [II] may be the same as those exemplified for the compound [I].

Suitable salt of the compound [III] may be the same as those exemplified for the compound [I] and an acid addition salt such as an inorganic acid addition salt [e.g. hydrochloride, hydrobromide, sulfate, phosphate, etc.], an organic acid addition salt [e.g. formate, acetate, trifluoroacetate, methanesulfonate, benzenesulfonate, toluenesulfonate, etc.] or the like.

This reaction is usually carried out in a conventional solvent such as alcohol (e.g. methanol, ethanol, etc.), dioxane, tetrahydrofuran, acetic acid, N,N-dimethylformamide, a mixture thereof or any other organic solvent which

does not adversely influence the reaction.

The reaction temperature is not critical, and the reaction is usually carried out under heating.

Process 2

The compound [Ic] or its salt can be prepared by reacting a compound [Ib] or its salt with an oxidizing agent.

Suitable salts of the compounds [Ib] and [Ic] may be the same as those exemplified for the compound [I].

The suitable oxidizing agent may be hydrogen peroxide, cumene hydroperoxide, tert-butyl hydroperoxide, Jones reagent, peracid [e.g. peracetic acid, perbenzoic acid, m-chloroperbenzoic acid, etc.], chromic acid, potassium permanganate, alkali metal periodate [e.g. sodium periodate, etc.] and the like.

This reaction is usually carried out in a solvent which does not adversely influence the reaction such as acetic acid, dichloromethane, acetone, ethyl acetate, chloroform, water, an alcohol [e.g. methanol, ethanol, etc.], a mixture thereof or the like.

The reaction temperature is not critical, and the reaction is usually carried out under cooling to warming.

Process 3

The compound [Id] or its salt can be prepared by the following methods.

Namely, 1) the compound [IV] or its salt is firstly reacted with a nitrite compound, and then 2) the resultant product is reacted with cuprous halide.

Suitable salt of the compound [Id] may be the same as those exemplified for the compound [I].

Suitable salt of the compound [IV] may be the same as chose exemplified for the compound [III] in Process 1.

Suitable nitrite compound may be alkali metal nitrite [e.g. sodium nitrite, potassium nitrite, etc.], alkyl nitrite [e.g. tert-butyl nitrite, etc.] and the like.

Suitable cuprous halide may be cuprous chloride, cuprous bromide and the like.

In the first step, the reaction is preferably carried out in the presence of an acid [e.g. sulfuric acid, etc.].

The reaction is usually carried out in a solvent such as water, tetrahydrofuran, dioxane, acetonitrile or any other organic solvent which does not adversely influence the reaction, or a mixture thereof.

The reaction temperature is not critical and the reaction can be carried out under cooling to warming.

In the second step, the reaction is preferably carried out in the presence of alkali metal halide [e.g. sodium bromide, etc.] and an inorganic acid [e.g. hydrobromic acid, etc.].

The reaction is usually carried out in a solvent such as water, tetrahydrofuran, dioxane or any other organic solvent which does not adversely influence the reaction.

The reaction temperature is not critical and the reaction can be carried out warming to heating.

Process 4

The compound [I2] or its salt can be prepared by reacting a compound [Ik] or its salt with a dehydrating agent.

Suitable salts of the compounds [Ik] and [Iℓ] may be the same as those exemplified for the compound [I].

Suitable dehydrating agent may be phosphorus compound [e.g. phosphorus pentoxide, phosphorus pentachloride, phosphorus oxychloride, etc.], thionyl chloride, acid anhydride [e.g. acetic anhydride, etc.], phosgene, arylsulphonyl chloride [e.g. benzenesulfonyl chloride, p-toluenesulfonyl chloride, etc.], methanesulfonyl chloride, sulfamic acid, ammonium sulfamate, N,N'-dicyclohexylcarbodiimide, lower alkoxycarbonyl halide [e.g. ethyl chloroformate, etc.] and the like.

The reaction is usually carried out in a conventional solvent such as acetonitrile, methylene chloride, ethylene chloride, benzene, N,N-dimethylformamide, pyridine or any other organic solvent which does not adversely influence the reaction.

Additionally in case that the above-mentioned dehydrating agents are in liquid, they can also be used as a solvent.

The reaction temperature is not critical and the reaction is preferably carried out under warming or heating.

Process 5

The compound [Im] can be prepared by the following methods.

Namely, 1) the compound [Ie] or its reactive derivative at the carboxy group or a salt thereof is firstly reacted with a compound [V], and then 2) subjecting the resultant product to hydrolysis reaction.

Suitable salts of the compound [Ie] and its reactive derivative at the carboxy group may be the same as those exemplified for the compound [I].

Suitable reactive derivative at the carboxy group of the compound [Ie] may be an acid halide [e.g. acid chloride, acid bromide, etc.].
In the first step, the reaction is preferably carried out in the presence of a base such as an alkali metal [e.g. lithium, sodium, potassium, etc.], alkaline earth metal [e.g. calcium, magnesium, etc.], alkali metal hydride [e.g. sodium hydride, etc.], alkaline earth metal hydride [e.g. calcium hydride, etc.], alkali metal alkoxide [e.g. sodium methoxide, sodium ethoxide, potassium tert-butoxide, etc.], alkaline earth metal alkoxide [e.g. magnesium methoxide and magnesium ethoxide, etc.].
The reaction is usually carried out in a solvent which does not adversely influence the reaction such as diethyl ether, tetrahydrofuran, and dioxane.
The reaction temperature is not critical and the reaction can be carried out under cooling to heating.
In this reaction, a compound of the formula :

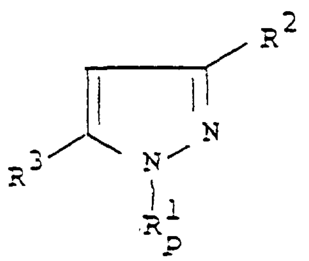

[Iy]
or its salt

wherein

| | |
|---|---|
| $R^2$ and $R^3$ | are each as defined above and |
| $R^1_p$ | is phenyl substituted with di($C_1$-$C_6$-alkoxycarbonyl)acetyl, may be obtained. |

Suitable salt of the compound [Iy] may be the same as those exemplified for the compound [I].
The compound [Iy] or its salt is further subjected to hydrolysis to give the compound [Im].
The hydrolysis is preferably carried out in the presence of an acid.
Suitable acid may be an organic acid [e.g. formic acid, acetic acid, propionic acid, trifluoroacetic acid, trichloroacetic acid, etc.], an inorganic acid [e.g. hydrochloric acid, hydrobromic acid, hydroiodic acid, sulfuric acid, etc.] and Lewis acid [e.g. boron tribromide, etc.].
The reaction is usually carried out in a solvent such as water, an alcohol [e.g. methanol, ethanol, etc.], methylene chloride, tetrahydrofuran, a mixture thereof or any other solvent which does not adversely influence the reaction. A liquid base or acid can be also used as the solvent. The reaction temperature is not critical and the reaction is usually carried out under cooling to'heating.

<u>Process 6</u>

The compound [Ir] or its salt can be prepared by reacting a compound [Iq] or its salt with an alkylating agent.
Suitable salts of the compounds [Iq] and [Ir] may be the same as those exemplified for the compound [I].
Suitable alkylating agent may be $C_1$-$C_6$ alkyl halide [e.g. methyl iodide, ethyl bromide, etc.].
The reaction is preferably carried out in the presence of a base such as an alkali metal [e.g. sodium, potassium, etc.], an alkaline earth metal [e.g. magnesium, calcium, etc.], the hydride or hydroxide or carbonate or bicarbonate thereof.
The reaction is usually carried out in a conventional solvent which does not adversely influence the reaction such as water, dioxane, an alcohol [e.g. methanol, ethanol, etc.], acetonitrile, tetrahydrofuran, N,N-dimethylformamide, or a mixture thereof.
Additionally, in case that the above-mentioned alkylating agents are in liquid, they can also be used as a solvent.
The reaction temperature is not critical and the reaction can be carried out under cooling to heating.
The compounds obtained by the above processes can be isolated and purified by a conventional method such as pulverization, recrystallization, column chromatography, reprecipitation, or the like.
It is to be noted that the compound [I] and the other compounds may include one or more stereoisomers due to asymmetric carbon atoms, and all of such isomers and mixture thereof are included within the scope of this invention.
The object compound [I] or its pharmaceutically acceptable salts thereof possesses strong antiinflammatory, analgesic and antithrombotic activities, and are useful for the treatment and/or prevention of inflammatory conditions,

various pains, collagen diseases, autoimmune diseases, various immunity diseases and thrombosis in human beings or animals, and more particularly for the treatment and/or prevention of inflammation and pain in joint and muscle [e.g. rheumatoid arthritis, rheumatoid spondylitis, osteoarthritis, gouty arthritis, etc.], inflammatory skin condition [e.g. sunburn, eczema, etc.], inflammatory eye condition [e.g. conjunctivitis etc.], lung disorder in which inflammation is involved [e.g. asthma, bronchitis, pigeon fancier's disease, farmer's lung, etc.], condition of the gastrointestinal tract associated with inflammation [e.g. aphthous ulcer, Crohn's disease, atropic gastritis, gastritis varialoforme, ulcerative colitis, coeliac disease, regional ileitis, irritable bowel syndrome, etc.], gingivitis, inflammation, pain and tumescence after operation or injury, pyresis, pain and other conditions associated with inflammation, particularly those in which lipoxygenase and cyclooxygenase products are a factor, systemic lupus erythematosus, scleroderma, polymyositis, periarteritis nodosa, rheumatic fever, Sjögren's syndrome, Behcet disease, thyroiditis, type I diabetes, nephrotic syndrome, aplastic anemia, myasthenia gravis, uveitis, contact dermatitis, psoriasis, Kawasaki disease, sarcoidosis, Hodgkin's disease and the like.
Additionally, the object compound is expected to be useful as therapeutical and/or preventive agents for cardiovascular or cerebrovascular diseases, the diseases caused by hyperglycemia and hyperlipemia.

In order to illustrate the usefulness of the object compound [I], the pharmacological test data of the compound [I] are shown in the following.

[A] ANTIINFLAMMATORY ACTIVITY ;

Effect on adjuvant arthritis in rats :

(i) Test Method :

Ten female Sprague-Dawley rats were used per group. A dose of 0.5 mg of Mycobacterium tuberculosis (strain Aoyama B) suspended in 0.05 ml of liquid paraffin was injected subcutaneously in the right hind paw. The injection of mycobacterial adjuvant produced local inflammatory lesions (primary lesion) and then about 10 days later, secondary lesions in both the injected and uninjected paws. The difference in volumes of both paws before and after adjuvant injection was the measure of arthritis. The drug was given orally once a day for 23 consecutive days from day 1.

(ii) Test Results :

| Test compound (Example No.) | Dose (mg/kg) | Inhibition of secondary lesion (uninjected paw) (%) |
|---|---|---|
| 1 | 3.2 | 93.6 |
| 2 | 3.2 | 89.3 |
| 3 | 3.2 | 99.1 |
| 4 | 3.2 | 81.0 |
| 7 | 3.2 | 100 |
| 8-4) | 3.2 | 95.7 |
| 11-2) | 3.2 | 95.6 |
| 16-1) | 3.2 | 100 |
| 16-2) | 3.2 | 100 |
| Ibuprofen | 100 | 79.6 |

[B] ANALGESIC ACTIVITY :

Inflammatory hyperalgesia induced by brewer's yeast in rats :

(i) Test Method :

Ten male Sprague Dawley rats were used per group. 0.1 ml of 5% brewer's yeast suspended in 0.5% methylcellulose was injected into the right hind paw. The pain threshold was determined 3 hours after yeast injection, by

applying pressure to the foot and reading the pressure at which the rat withdrew the foot.
The drugs were given orally 2 hours after yeast injection. The pain threshold in the treated animals was compared with that in the control animals.

(ii) Test Results :

| Test compound (Example No.) | Dose (mg/kg) | Relative potency (Control=1.0) |
|---|---|---|
| 1 | 10 | 1.36 |
| 2 | 10 | 1.38 |
| 3 | 10 | 1.47 |
| 9 | 10 | 1.53 |

[C] ANTI-RHEUMATIC ACTIVITY :

Effect on collagen induced arthritis in mice :

(i) Test Method :

Eight male DBA/l mice were used per group. Type II bovine collagen was solubilized in 0.1 M acetic acid and emulsified in complete Freund's adjuvant (CFA). Mice were primed with 0.2 mg of Type II collagen in CFA intradermally at the base of the tail. Mice were challenged after 21 day with the same procedure. From 10 day after challenge, drug was administered orally once a day for 3 weeks and mice were inspected weekly for visual signs of arthritis. An arthritis index was used to grade limb 0-3, representing joint swelling and erythema (Grade 1), visible joint disorder (Grade 2) and detectable joint ankylosis (Grade 3).

(ii) Test Results :

| Test compound (Example No.) | Dose (mg/kg) | Inhibition of arthritis index (%) |
|---|---|---|
| 1 | 10 | 51.3 |
| 2 | 10 | 61.7 |
| 7 | 10 | 83.3 |

For therapeutic purpose, the compound [I] and a pharmaceutically acceptable salt thereof of the present invention can be used in a form of pharmaceutical preparation containing one of said compounds as an active ingredient, in admixture with a pharmaceutically acceptable carrier such as an organic or inorganic solid or liquid excipient suitable for oral, parenteral or external (topical) administration. The pharmaceutical preparations may be capsules, tablets, dragees, granules, inhalant, suppositories, solution, lotion, suspension, emulsion, ointment, gel, or the like. If desired, there may be included in these preparations, auxiliary substances, stabilizing agents, wetting or emulsifying agents, buffers and other commonly used additives.
While the dosage of the compound [I] will vary depending upon the age and condition of the patient, an average single dose of about 0.1 mg, 1 mg, 10 mg, 50 mg, 100 mg, 250 mg, 500 mg and 1000 mg of the compound [I] may be effective for treating the above-mentioned diseases.
In general, amounts between 0.1 mg/body and about 1,000 mg/body may be administered per day.
The following Preparations and Examples are given for the purpose of illustrating this invention.

Preparation 1

A mixture of ethyl 1-(4-methoxyphenyl)-5-[4-(methylthio)phenyl]pyrazole-3-carboxylate (10.2 g) and potassium hydroxide (3.7 g) in methanol (60 ml) was refluxed for 30 minutes. The solvent was evaporated, and the residue was dissolved in water and washed with toluene. The aqueous layer was acidified, and the precipitates were collected

and washed with ethyl acetate to give crystals of 1-(4-methoxyphenyl)-5-[4-(methylthio)-phenyl]pyrazole-3-carboxylic acid (8.8 g).

| | |
|---|---|
| mp : | 238-239°C (dec.) |
| IR (Nujol) : | 2750, 2600, 1700, 1600, 1510 $cm^{-1}$ |
| Mass (m/z) : | 340 ($M^+$). |

Preparation 2

A mixture of 1-(4-methoxyphenyl)-5-[4-(methylthio)-phenyl]pyrazole-3-carboxylic acid (3.4 g) and phosphorus pentachloride (2.3 g) in dichloromethane (20 ml) was stirred at ambient temperature for 1 hour. The solvent was evaporated to give an oil of the corresponding acid chloride.

A solution of the obtained oil in acetone (10 ml) was added dropwise to an ice-cooled mixture of sodium azide (0.65 g), water (10 ml) and acetone (10 ml). During the addition of the above solution, the reaction mixture was kept at pH 8 to 9 by addition of an aqueous sodium bicarbonate solution. The mixture was stirred at ambient temperature for 30 minutes, concentrated in vacuo, and extracted with ethyl acetate. The extract was washed with water, dried, and concentrated to give an oil of the corresponding acid azide.

The above oil was dissolved in toluene (50 ml). The solution was heated at 100°C for 1 hour and concentrated to give a solid of the corresponding isocyanate.

A mixture of the above solid and concentrated hydrochloric acid (10 ml) in acetic acid (10 ml) was refluxed for 5 hours, and then concentrated. A solution of sodium hydroxide was added to the residue and the mixture was extracted with chloroform. The extract was washed with water, dried, and concentrated in vacuo. The residue was purified by column chromatography on silica gel eluting with chloroform and the purified product was recrystallized from ethanol to give crystals of 1-(4-methoxyphenyl)-5-[4-(methylthio)phenyl]pyrazol-3-amine (0.8 g).

| | |
|---|---|
| mp : | 120-129°C |
| IR (Nujol) : | 3450, 3300, 3200, 1630, 1610, 1565, 1520 $cm^{-1}$ |
| NMR ($CDCl_3$, δ) : | 2.46 (3H, s), 3.32 (2H, broad s), 3.80 (3H, s), 5.87 (1H, s), 6.7-7.2 (8H, m) |
| Mass (m/z) : | 311 ($M^+$) |

Example 1

A mixture of 4,4-difluoro-1-[4-(methylthio)phenyl]-butane-1,3-dione (2.4 g) and 4-methoxyphenylhydrazine hydrochloride (1.9 g) in acetic acid (10 ml) was stirred at 100°C for 1 hour. The solvent was evaporated and the residue was purified by column chromatography on silica gel (30 g) eluting with toluene. The purified oily product was crystallized from ethanol to give crystals of 3-(difluoromethyl)-1-(4-methoxyphenyl)-5-[4-(methylthio)-phenyl]pyrazole (2.5 g).

| | |
|---|---|
| mp : | 100-101°C |
| IR (Nujol) : | 1610, 1520, 1500 $cm^{-1}$ |
| NMR ($CDCl_3$, δ) : | 2.48 (3H, s), 3.82 (3H, s), 6.5-7.3 (10H, m) |
| Mass (m/z) : | 346 ($M^+$) |

Example 2

A mixture of 3-(difluoromethyl)-1-(4-methoxyphenyl)-5-[4-(methylthio)phenyl]pyrazole (1 g) and m-chloroperbenzoic acid (0.56 g) in dichloromethane (23 ml) was stirred at ambient temperature for 1 hour. The mixture was washed with an aqueous solution of sodium bicarbonate, dried over magnesium sulfate, and concentrated. The oily residue was crystallized from ethanol to give crystals of 3-(difluoromethyl)-1-(4-methoxyphenyl)-5-[4-(methylsulfinyl)phenyl] pyrazole (0.82 g).

| | |
|---|---|
| mp : | 123-124°C |
| IR (Nujol) : | 1615, 1590, 1520 $cm^{-1}$ |
| NMR ($CDCl_3$, δ) : | 2.74 (3H, s), 3.83 (3H, s), 6.5-7.7 (10H, m) |

Example 3

A mixture of 3-(difluoromethyl)-1-(4-methoxyphenyl)-5-[4-(methylthio)phenyl]pyrazole (2 g), 30% hydrogen peroxide (2 ml) and sulfuric acid (2 drops) in acetic acid (5 ml) was stirred at 60°C for 1 hour. The solvent was evaporated

and the residue was recrystallized from ethyl acetate to give crystals of 3-(difluoromethyl)-1-(4-methoxyphenyl)-5-[4-(methylsulfonyl)phenyl]pyrazole (0.86 g).

mp : 135°C
IR (Nujol) : 1605, 1520 $cm^{-1}$
NMR ($CDCl_3$, δ) : 3.07 (3H, s), 3.84 (3H, s), 6.1-8.0 (10H, m)
Mass (m/z) : 378 ($M^+$), 346

Example 4

A solution of sodium nitrite (492 mg) in water (0.6 ml) was added dropwise to a mixture of 1-(4-methoxyphenyl)-5-[4-(methylthio)phenyl]pyrazol-3-amine (2 g) and sulfuric acid (1.2 ml) in water (3 ml) and acetonitrile (6 ml) at 5°C, and the mixture was stirred at the same temperature for 1 hour. The mixture was added portionwise to a mixture of cuprous bromide (1.25 g), sodium bromide (1.2 g) and hydrobromic acid (3.3 ml) in water (6 ml) at 70 to 80°C during a period of 15 minutes, and the resulting mixture was stirred at 80°C for 30 minutes. The aqueous layer was removed by decantation and the oily residue was extracted with toluene. The extract was washed with water, dried, and concentrated in vacuo. The residue (1.1 g) was purified by column chromatography on silica gel (20 g) eluting with a mixture of chloroform and n-hexane (2:1) to give crystals of 3-bromo-1-(4-methoxyphenyl)-5-[4-(methylthio)phenyl] pyrazole (0.62 g).

mp : 144-145°C
IR (Nujol) : 1605, 1510 $cm^{-1}$
NMR ($CDCl_3$, δ) : 2.47 (3H, s), 3.81 (3H, s), 6.47 (1H, s), 6.8-7.3 (8H, m)
Mass (m/z) : 376 ($M^+$)

Example 5

A mixture of 1-[4-(methylthio)phenyl]-4,4,4-trifluorobutane-1,3-dione (1.8 g) and 4-cyanophenylhydrazine hydrochloride (1.2 g) in acetic acid (30 ml) and N,N-dimethylformamide (1 ml) was stirred at 110°C for 3 hours. The reaction mixture was poured into water and extracted with ethyl acetate. The extract was washed with water, dried over anhydrous magnesium sulfate, and evaporated to give a brown powder of 1-(4-cyanophenyl)-5-[4-(methylthio) phenyl]-3-(trifluoromethyl)pyrazole (2.5 g).

mp : 145-147°C
IR (Nujol) : 2230, 1610, 1595, 1515 $cm^{-1}$
NMR ($CDCl_3$, δ) : 2.50 (3H, s), 6.75 (1H, s), 7.0-7.8 (8H, m)
Mass (m/z) : 359 ($M^+$)

Example 6

The following compounds were obtained according to a similar manner to that of Example 5.

1) 3-(Difluoromethyl)-1-[3,4-(methylenedioxy)phenyl]-5-[4-(methylthio)phenyl]pyrazole

IR (Film) : 1605, 1520, 1485 $cm^{-1}$
NMR ($CDCl_3$, δ) : 2.48 (3H, s), 5.99 (2H, s), 6.6-7.3 (9H, m)
Mass (m/z) : 360 ($M^+$)

2) 1-[3,4-(Methylenedioxy)phenyl]-5-[4-(methylthio)-phenyl]-3-(trifluoromethyl)pyrazole

IR (Film) : 1590, 1490 $cm^{-1}$
NMR ($CDCl_3$, δ) : 2.48 (3H, s), 6.02 (2H, s), 6.6-7.3 (8H, m)
Mass (m/z) : 378 ($M^+$)

Example 7

A mixture of 1-(4-cyanophenyl)-5-[4-(methylthio)-phenyl)-3-(trifluoromethyl)pyrazole (2.5 g) and m-chloroperbenzoic acid (3.2 g) in dichloromethane (100 ml) was stirred at ambient temperature for 2 hours. The mixture was

washed with an aqueous solution of sodium bicarbonate, dried, and concentrated. The oily residue (3 g) was purified by column chromatography on silica gel eluting with a mixture of chloroform and methanol (40:1). The purified product (2.1 g) was recrystallized from a mixture of isopropanol and ethanol to give yellow crystals of 1-(4-cyanophenyl)-5-[4-(methylsulfonyl)phenyl]-3-(trifluoromethyl)pyrazole (1.3 g).

| | |
|---|---|
| mp : | 140-142°C |
| IR (Nujol) : | 2230, 1610, 1515, 1500 $cm^{-1}$ |
| NMR ($CDCl_3$, δ) : | 3.11 (3H, s), 6.89 (1H, s), 7.4-8.0 (8H, m) |
| Mass (m/z) : | 391 ($M^+$) |

Example 8

The following compounds were obtained according to a similar manner to that of Example 7.

1) 3-(Difluoromethyl)-1-[3,4-(methylenedioxy)phenyl]-5-[4-(methylsulfonyl)phenyl]pyrazole

| | |
|---|---|
| mp : | 120-121°C |
| IR (Nujol) : | 1600, 1505, 1490 $cm^{-1}$ |
| NMR ($CDCl_3$, δ) : | 3.08 (3H, s), 6.05 (2H, s), 6.5-7.1 (5H, m), 7.45 (2H, d, J=8Hz), 7.91 (2H, d, J=8Hz) |
| Mass (m/z) : | 392 ($M^+$) |

2) 1-(4-Cyanophenyl)-3-(difluoromethyl)-5-[4-methylsulfonyl)phenyl]pyrazole

| | |
|---|---|
| mp : | 140-141°C |
| IR (Nujol) : | 2230, 1610, 1515 $cm^{-1}$ |
| NMR ($CDCl_3$, δ) : | 3.11 (3H, s), 6.5-7.5 (6H, m), 7.70 (2H, d, J=8Hz), 7.96 (2H, d, J=8Hz) |
| Mass (m/z) : | 373 ($M^+$) |

3) 1-(4-Acetylphenyl)-3-(difluoromethyl)-5-[4-(methylsulfonyl)phenyl]pyrazole

| | |
|---|---|
| mp : | 121-122°C |
| IR (Nujol) : | 1685, 1600, 1515 $cm^{-1}$ |
| NMR ($CDCl_3$, δ) : | 2.63 (3H, s), 3.10 (3H, s), 6.5-7.5 (6H, m), 7.9-8.1 (4H, m) |
| Mass (m/z) : | 390 ($M^+$), 375 |

4) 1-(4-Acetylphenyl)-5-[4-(methylsulfonyl)phenyl]-3-(trifluoromethyl)pyrazole

| | |
|---|---|
| mp : | 120-122°C |
| IR (Nujol) : | 1690, 1605, 1500 $cm^{-1}$ |
| NMR ($CDCl_3$, δ) : | 2.63 (3H, s), 3.10 (3H, s), 6.64 (1H, s), 7.1-8.0 (8H, m) |
| Mass (m/z) : | 408 ($M^+$) |

5) 1-[3,4-(Methylenedioxy)phenyl]-5-[4-(methylsulfonyl)-phenyl]-3-(trifluoromethyl)pyrazole

mp : 110-111°C

| | |
|---|---|
| IR (Nujol) : | 1605, 1510 $cm^{-1}$ |
| NMR ($CDCl_3$, δ) : | 3.09 (3H, s), 6.06 (2H, s), 6.6-6.9 (4H, m), 7.45 (2H, d, J=8Hz), 7.92 (2H, d, J=8Hz) |
| Mass (m/z) : | 410 ($M^+$) |

6) 1-(4-Methoxyphenyl)-5-[4-(methylsulfonyl)phenyl]-3-(trifluoromethyl)pyrazole

| | |
|---|---|
| mp : | 155-156°C |
| IR (Nujol) : | 1600, 1500 $cm^{-1}$ |
| NMR (DMSO-$d_6$, δ) : | 3.25 (3H, s), 3.80 (3H, s), 7.03 (2H, d, J=8Hz), 7.2-7.6 (5H, m), 7.93 (2H, d, J=8Hz) |
| Mass (m/z) : | 396 ($M^+$) |

7) 3-(Chloromethyl)-1-(4-methoxyphenyl)-5-[4-(methylsulfonyl)phenyl]pyrazole

mp : 115-116°C
IR (Nujol) : 1600, 1510 $cm^{-1}$
NMR ($CDCl_3$, δ) : 3.07 (3H, s), 3.83 (3H, s), 4.70 (2H, s), 6.68 (1H, s), 6.8-7.9 (8H, m)
Mass (m/z) : 376 ($M^+$)

8) 3-(Difluoromethyl)-1-(2-methoxyphenyl)-5-[4-(methylsulfonyl)phenyl]pyrazole

mp : 152-153°C
IR (Nujol) : 1600, 1510, 1500 $cm^{-1}$
NMR ($CDCl_3$, δ) : 3.04 (3H, s), 3.46 (3H, s), 6.5-7.5 (8H, m), 7.85 (2H, d, J=8Hz)
Mass (m/z) : 378 ($M^+$)

9) 1-(4-Methoxyphenyl)-5-[2-(methylsulfonyl)phenyl]-3-(trifluoromethyl)pyrazole

mp : 128-129°C
IR (Nujol) : 1615, 1590, 1520, 1495 $cm^{-1}$
NMR ($CDCl_3$, δ) : 2.87 (3H, s), 3.75 (3H, s), 6.7-6.9 (3H, m), 7.2-8.2 (6H, m)
Mass (m/z) : 396 ($M^+$)

10) 3-(Difluoromethyl)-1-(4-methoxyphenyl)-5-[2-(methylsulfonyl)phenyl]pyrazole

mp : 124-125°C
IR (Nujol) : 1615, 1595, 1520, 1495 $cm^{-1}$
NMR ($CDCl_3$, δ) : 2.90 (3H, s), 3.75 (3H, s), 6.5-7.4 (7H, m), 7.5-8.2 (3H, m)
Mass (m/z) : 378 ($M^+$)

Example 9

A mixture of 3-(difluoromethyl)-1-[3,4-(methylenedioxy)phenyl]-5-[4-(methylthio)phenyl]pyrazole (2.7 g) and m-chloroperbenzoic acid (1.8 g) in dichloromethane (80 ml) was stirred at 5°C for 4 hours. The mixture was washed with an aqueous solution of sodium bicarbonate, dried, and concentrated. The residue (2.9 g) was purified by column chromatography on silica gel eluting with a mixture of chloroform and methanol (25:1). The purified product (1.3 g) was recrystallized from isopropanol to give 3-(difluoromethyl)-1-[3,4-(methylenedioxy)phenyl]-5-[4-(methylsulfinyl)phenyl]pyrazole (0.85 g).

mp : 104-105°C
IR (Nujol) : 1610, 1510, 1490 $cm^{-1}$
NMR (DMSO-$d_6$, δ) : 2.77 (3H, s), 6.12 (2H, s), 6.7-7.8 (9H, m)
Mass (m/z) : 376 ($M^+$), 359

Example 10

A solution of phosphorus oxychloride (1.7 g) in N,N-dimethylformamide (20 ml) was stirred at 5°C for 30 minutes. To the solution was added 1-(4-carbamoylphenyl)-3-(difluoromethyl)-5-[4-(methylsulfonyl)phenyl]pyrazole (2 g). The resulting mixture was stirred at 5°C for 2 hours, poured into ice-water, and extracted with a mixture of tetrahydrofuran and ethyl acetate. The extract was washed with water, dried, and evaporated to give an oil of 1-(4-cyanophenyl)-3-(difluoromethyl)-5-[4-(methylthio)-phenyl]pyrazole (2.0 g).

IR (Nujol) : 2240, 1610, 1515, 1500 $cm^{-1}$
NMR ($CDCl_3$, δ) : 2.50 (3H, s), 6.5-7.7 (10H, m)
Mass (m/z) : 341 ($M^+$)

Example 11

The following compounds were obtained according to a similar manner to that of Example 10.

1) 1-(4-Cyanophenyl)-5-[4-(methylsulfonyl)phenyl]-pyrazole-3-carbonitrile

mp : 159-160°C
IR (Nujol) : 2250, 2240, 1610, 1550, 1505 $cm^{-1}$
NMR ($CDCl_3$, δ) : 3.11 (3H, s), 7.01 (1H, s), 7.4-8.0 (8H, m)
Mass (m/z) : 348 ($M^+$)

2) 1-(4-Acetylphenyl)-5-[4-(methylsulfonyl)phenyl]-pyrazole-3-carbonitrile

mp : 155-156°C
IR (Nujol) : 2250, 1695, 1605, 1510 $cm^{-1}$
NMR ($CDCl_3$, δ) : 2.64 (3H, s), 3.10 (3H, s), 6.99 (1H, s), 7.1-8.1 (8H, m)
Mass (m/z) : 365 ($M^+$), 350

3) 1-[3,4-(Methylenedioxy)phenyl]-5-[4-(methylsulfonyl)-phenyl]pyrazole-3-carbonitrile

mp : 182-183°C
IR (Nujol) : 2250, 1605, 1495 $cm^{-1}$
NMR ($CDCl_3$, δ) : 3.09 (3H, s), 6.07 (2H, s), 6.6-6.9 (3H, m), 6.94 (1H, s), 7.43 (2H, d, J=8Hz), 7.93 (2H, d, J=8Hz)
Mass (m/z) : 367 ($M^+$)

Example 12

A mixture of 1-(4-carboxyphenyl)-3-(difluoromethyl)-5-[4-(methylthio)phenyl]pyrazole (2 g) and thionyl chloride (20 ml) was refluxed for 3 hours, and concentrated in vacuo, giving 1-[4-(chloroformyl)phenyl]-3-(difluoromethyl)-5-[4-(methylthio)phenyl]pyrazole.
A solution of diethyl malonate (1.4 g) and ethanol (0.3 ml) in diethyl ether (15 ml) was added dropwise to a stirred mixture of magnesium (0.16 g), ethanol (0.2 ml) and carbon tetrachloride (0.36 ml) in diethyl ether (5 ml). The resulting mixture was refluxed for 1 hour. A solution of the above acid chloride in tetrahydrofuran (15 ml) was added dropwise to the mixture. The mixture was stirred at ambient temperature for 1 hour and refluxed for 1 hour. The reaction mixture was poured into diluted hydrochloric acid and extracted with ethyl acetate. The extract was washed with water, dried, and concentrated.
A mixture of the residue (4 g), acetic acid (40 ml), and 20% sulfuric acid (20 ml) was refluxed for 3.5 hours and then concentrated. The residue was dissolved in ethyl acetate, and the solution was washed with water, dried, and concentrated. The residue was purified by column chromatography on silica gel eluting with a mixture of toluene and ethyl acetate (4:1) to give crystals of 1-(4-acetylphenyl)-3-(difluoromethyl)-5-[4-(methylthio)-phenyl]pyrazole (0.4 g).

mp : 143-145°C
IR (Nujol) : 1680, 1600 $cm^{-1}$
NMR ($CDCl_3$, δ) : 2.49 (3H, s), 2.61 (3H, s), 6.5-7.5 (8H, m), 7.95 (2H, d, J=7Hz)
Mass (m/z) : 358 ($M^+$)

Example 13

The following compounds were obtained according to a similar manner to that of Example 12.

1) 1-(4-Acetylphenyl)-5-[4-(methylthio)phenyl]-3-(trifluoromethyl)pyrazole

IR (Film) : 1690, 1610, 1500 $cm^{-1}$
NMR ($CDCl_3$, δ) : 2.49 (3H, s), 2.61 (3H, s), 6.75 (1H, s), 7.1-8.2 (8H, m)
Mass (m/z) : 376 ($M^+$)

**Comparative Example 1**

Sodium hydride (0.17 g) was added to a solution of 1-(4-hydroxyphenyl)-5-[4-(methylsulfonyl)phenyl]-3-(trifluoromethyl)pyrazole (1.5 g) in N,N-dimethylformamide (15 ml) at 5°C. The mixture was stirred at ambient temperature for 30 minutes. To the mixture was added chloromethyl methyl ether (0.4 g) in N,N-dimethylformamide (2 ml) at 5°C. The obtained mixture was stirred at 5°C for 2 hours and at ambient temperature for 1 hour, poured into a mixture of ice and dilute hydrochloric acid, and extracted with ethyl acetate. The extract was washed with water, dried, and evaporated. The residue (1.3 g) was recrystallized from isopropanol to give crystals of 1-[4-(methoxymethoxy)phenyl]-5-[4-(methylsulfonyl)phenyl]-3-(trifluoromethyl)pyrazole (1.3 g).

| | |
|---|---|
| mp : | 96-97°C |
| IR (Nujol) : | 1610, 1520, 1500 $cm^{-1}$ |
| NMR ($CDCl_3$, δ) : | 3.08 (3H, s), 3.49 (3H, s), 5.20 (2H, s), 6.84 (1H, s), 7.0-8.0 (8H, m) |
| Mass (m/z) : | 426 ($M^+$) |

Example 14

The following compounds were obtained according to a similar manner to that of Comparative Example 1.

1) 1-(4-Ethoxyphenyl)-5-[4-(methylsulfonyl)phenyl]-3-(trifluoromethyl)pyrazole

| | |
|---|---|
| mp : | 140°C |
| IR (Nujol) : | 1610, 1600, 1520, 1500 $cm^{-1}$ |
| NMR ($CDCl_3$, δ) : | 1.43 (3H, t, J=7Hz), 3.07 (3H, s), 4.05 (2H, q, J=7Hz), 6.8-7.5 (7H, m), 7.91 (2H, d, J=8Hz) |
| Mass (m/z) : | 410 ($M^+$) |

2) 1-(4-Isopropoxyphenyl)-5-[4-(methylsulfonyl)phenyl]-3-(trifluoromethyl)pyrazole

| | |
|---|---|
| mp : | 99-100°C |
| IR (Nujol) : | 1610, 1525, 1510 $cm^{-1}$ |
| NMR ($CDCl_3$, δ) : | 1.35 (6H, d, J=6Hz), 3.07 (3H, s), 4.4-4.7 (1H, m), 6.8-7.5 (7H, m), 7.90 (2H, d, J=8Hz) |
| Mass (m/z) : | 424 ($M^+$) |

3) 3-(Difluoromethyl)-1-(4-ethoxyphenyl)-5-[4-(methylsulfonyl)phenyl]pyrazole

| | |
|---|---|
| mp : | 105-107°C |
| IR (Nujol) : | 1605, 1520, 1500 $cm^{-1}$ |
| NMR ($CDCl_3$, δ) : | 1.43 (3H, t, J=7Hz), 3.07 (3H, s), 4.05 (2H, q, J=7Hz), 6.5-7.5 (8H, m), 7.89 (2H, d, J=8Hz) |
| Mass (m/z) : | 392 ($M^+$) |

4) 3-(Difluoromethyl)-1-(4-isopropoxyphenyl)-5-[4-(methylsulfonyl)phenyl]pyrazole

| | |
|---|---|
| mp : | 90°C |
| IR (Nujol) : | 1605, 1515, 1500 $cm^{-1}$ |
| NMR ($CDCl_3$, δ) : | 1.35 (6H, d, J=6Hz), 3.07 (3H, s), 4.4-4.7 (1H, m), 6.5-7.5 (8H, m), 7.89 (2H, d, J=8Hz) |
| Mass (m/z) : | 406 ($M^+$) |

Example 15

The following compounds were obtained according to a similar manner to that of Example 1.

1) 1-(4-Methoxyphenyl)-5-[4-(methylthio)phenyl]-3-trifluoromethyl)pyrazole

| | |
|---|---|
| mp : | 98-100°C |
| IR (Nujol) : | 1605, 1520, 1500 $cm^{-1}$ |
| NMR ($CDCl_3$, δ) : | 2.48 (3H, s), 3.82 (3H, s), 6.71 (1H, s), 6.8-7.3 (8H, m) |
| Mass (m/z) : | 364 ($M^+$) |

2) 3-(Chloromethyl)-1-(4-methoxyphenyl)-5-[4-(methylthio)phenyl]pyrazole

| | |
|---|---|
| IR (Film) : | 1600, 1510 $cm^{-1}$ |
| NMR ($CDCl_3$, δ) : | 2.47 (3H, s), 3.82 (3H, s), 4.70 (2H, s), 6.5-7.7 (9H, m) |
| Mass (m/z) : | 344 ($M^+$) |

3) 3-(Difluoromethyl)-1-(2-methoxyphenyl)-5-[4-(methylthio)phenyl]pyrazole

| | |
|---|---|
| mp : | 98-100°C |
| IR (Nujol) : | 1605, 1515, 1500 $cm^{-1}$ |
| NMR ($CDCl_3$, δ) : | 2.44 (3H, s), 3.50 (3H, s), 6.4-7.5 (10H, m) |
| Mass (m/z) : | 346 ($M^+$) |

4) 1-(4-Methoxyphenyl)-5-[2-(methylthio)phenyl]-3-(trifluoromethyl)pyrazole

| | |
|---|---|
| mp : | 78-80°C |
| IR (Nujol) : | 1610, 1590, 1520, 1490 $cm^{-1}$ |
| NMR ($CDCl_3$, δ) : | 2.34 (3H, s), 3.77 (3H, s), 6.6-7.4 (9H, m) |
| Mass (m/z) : | 364 ($M^+$) |

5) 3-(Difluoromethyl)-1-(4-methoxyphenyl)-5-[2-(methylthio)phenyl]pyrazole

| | |
|---|---|
| mp : | 79-81°C |
| IR (Nujol) : | 1615, 1590, 1520, 1490 $cm^{-1}$ |
| NMR ($CDCl_3$, δ) : | 2.33 (3H, s), 3.76 (3H, s), 6.5-7.4 (10H, m) |
| Mass (m/z) : | 346 ($M^+$) |

Example 16

The following compounds were obtained according to a similar manner to that of Example 2.

1) 3-Bromo-1-(4-methoxyphenyl)-5-[4-(methylsulfinyl)-phenyl]pyrazole

| | |
|---|---|
| mp : | 140-142°C |
| IR (Nujol) : | 1610, 1585, 1515 $cm^{-1}$ |
| NMR (DMSO-$d_6$, δ) : | 2.75 (3H, s), 3.83 (3H, s), 6.60 (1H, s), 6.85-7.63 (8H, m) |
| Mass (m/z) : | 392, 390, 377 |

2) 1-(4-Cyanophenyl)-5-[4-(methylsulfinyl)phenyl]-3-(trifluoromethyl)pyrazole

| | |
|---|---|
| mp : | 121-122°C |
| IR (Nujol) : | 1605, 1510, 1495 $cm^{-1}$ |
| NMR ($CDCl_3$, δ) : | 2.78 (3H, s), 6.85 (1H, s), 7.3-7.8 (8H, m) |
| Mass (m/z) : | 375 ($M^+$) |

3) 1-(4-Acetylphenyl)-5-[4-(methylsulfinyl)phenyl]-3-(trifluoromethyl)pyrazole

| | |
|---|---|
| mp : | 145-146°C |
| IR (Nujol) : | 1690, 1600, 1500 $cm^{-1}$ |
| NMR ($CDCl_3$, δ) : | 2.62 (3H, s), 2.76 (3H, s), 6.84 (1H, s), 7.3-8.0 (8H, m) |
| Mass (m/z) : | 393 ($(M+1)^+$) |

Example 17

The following compounds were obtained according to a similar manner to that of Example 3.

1) 3-Bromo-1-(4-methoxyphenyl)-5-[4-(methylsulfonyl)-phenyl]pyrazole

| | |
|---|---|
| mp : | 177-178°C |
| IR (Nujol) : | 1610, 1515 $cm^{-1}$ |
| NMR (DMSO-$d_6$, δ) : | 3.13 (3H, s), 3.79 (3H, s), 6.97-7.93 (9H, m) |
| Mass (m/z) : | 408, 406 |

Example 18

To a solution of L-(+)-diethyl tartrate (3.71 g) in dichloromethane (75 ml) was added titanium (IV) isopropoxide

(2.7 ml) under the atmosphere of nitrogen. After being stirred for 5 minutes, water (162 µl) was added to the mixture. After the mixture was vigorously stirred for 25 minutes, 3-(difluoromethyl)-1-(4-methoxyphenyl)-5-[4-(methylthio)phenyl]pyrazole (6.24 g) was added to the mixture and the solution was cooled to -30°C and stirred for 40 minutes at -30°C to -20°C. To the mixture was added dropwise cumene hydroperoxide (3.00 ml). After an additional 10 minutes, the reaction mixture was sealed and allowed to stand overnight at -23°C. Water (3.03 ml) was added to the mixture and the mixture was stirred at ambient temperature for 90 minutes. The reaction mixture was filtered and to the filtrate were added 2N sodium hydroxide (48 ml) and brine (24 ml).
After being stirred for 1 hour at ambient temperature, the organic layer was separated, dried over magnesium sulfate and concentrated in vacuo. The residue was purified by column chromatography on silica gel (450 g) eluting with a mixture of dichloromethane and methanol (40:1). The obtained product was recrystallized eight times from a mixture of ethanol and isopropyl ether (1:4) to give R-(+)-3-(difluoromethyl)-1-(4-methoxyphenyl)-5-[4-(metylsulfinyl)phenyl] pyrazole (0.71 g).

| | |
|---|---|
| mp : | 113-114°C |
| $[\alpha]_D^{20}$ = | +69.4° (c=1.00, $CHCl_3$) |
| IR (Nujol) : | 1615, 1590, 1520 $cm^{-1}$ |
| NMR ($CDCl_3$, δ) : | 2.74 (3H, s), 3.83 (3H, s), 6.5-7.7 (10H, m) |
| Mass (m/z) : | 363 ($(M+1)^+$) |

Example 19

The following compound was obtained according to a similar manner to that of Example 18, except that D-(-)-diethyl tartrate was used.
S-(-)-3-(Difluoromethyl)-1-(4-methoxyphenyl)-5-[4-(methylsulfinyl)phenyl]pyrazole

| | |
|---|---|
| mp : | 112-114°C |
| $[\alpha]_D^{20}$ = | -70.9° (c=1.02, $CHCl_3$) |
| IR (Nujol) : | 1610, 1590, 1515 $cm^{-1}$ |
| NMR ($CDCl_3$, δ) : | 2.75 (3H, s), 3.83 (3H, s), 6.5-7.7 (10H, m) |
| Mass | (m/z) : 363 ($(M+1)^+$) |

**Claims**

**1.** A compound of the formula:

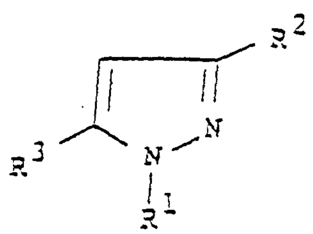

[I]

wherein

$R^1$ is phenyl which is substituted with substituent(s) selected from the group consisting of cyano; ($C_1$-$C_6$) alkylenedioxy ($C_1$-$C_6$)alkanoyl;
and ($C_1$-$C_6$)alkoxy;

$R^2$ is halogen; halo($C_1$-$C_6$)alkyl or cyano;

$R^3$ is phenyl substituted with ($C_1$-$C_6$)alkylthio; ($C_1$-$C_6$)alkylsulfinyl; or ($C_1$-$C_6$)alkylsulfonyl

provided that
when $R^1$ is phenyl substituted with ($C_1$-$C_6$)alkoxy then $R^2$ is halogen or halo($C_1$-$C_6$)alkyl and pharmaceutically

acceptable salts thereof.

**2.** A compound according to claim 1, wherein

$R^1$ is phenyl substituted with cyano, $C_1$-$C_6$ alkanoyl or $C_1$-$C_6$ alkoxy, and
R2 is halogen or halo ($C_1$-$C_6$)alkyl.

**3.** A process for preparing a compound according to claim 1 and pharmaceutically acceptable salts thereof, which comprises,

a) reacting a compound of the formula :

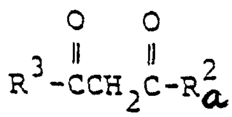

[II]

or its salt with a compound of the formula :

$$R^1\text{-NH-NH}_2 \qquad [III]$$

or its salt to provide a compound of the formula :

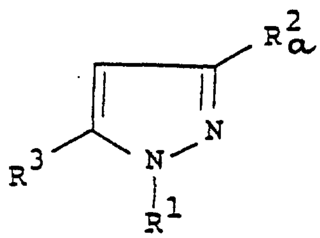

[Ia]

or its salt, in the above formulas,

$R^1$ and $R^3$ are each as defined in the present claim 1 and $R^2_a$ is halo($C_1$-$C_6$)alkyl or cyano, or

b) reacting a compound of the formula :

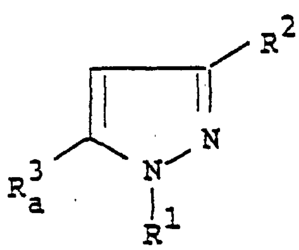

[Ib]

or its salt with an oxidizing agent to provide a compound of the formula :

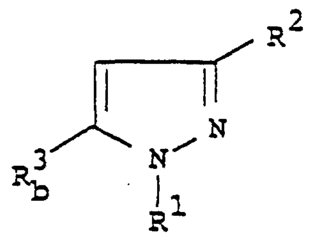

[Ic]

or its salt, in the above formulas,

$R^1$ and $R^2$ are each as defined in the present claim 1,
$R^3$ is phenyl substituted with ($C_1$-$C_6$)alkylthio, and
$R_b^3$ is phenyl substituted with ($C_1$-$C_6$)alkylsulfinyl or ($C_1$-$C_6$)alkylsulfonyl, or

c) reacting a compound of the formula :

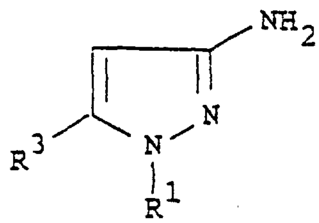

[IV]

or its salt with a nitrite compound,
and then reacting the resultant product with cuprous halide to provide a compound of the formula :

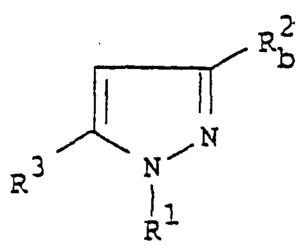

[Id]

or its salt, in the above formulas,

$R^1$ and $R^3$ are each as defined in the present claim 1, and
$R_b^2$ is halogen, or

d) reacting a compound of the formula :

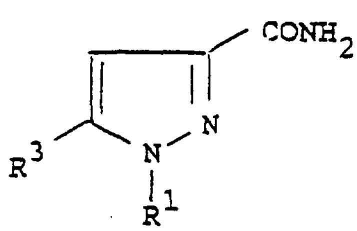

[Ik]

or its salt with a dehydrating agent to provide a compound of the formula :

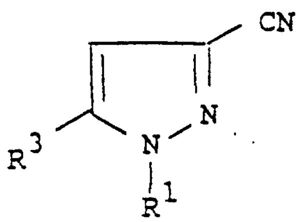

[Iℓ]

or its salt, in the above formulas.

$R^1$ and $R^3$ are each as defined in the present claim 1, or

e) reacting a compound of the formula :

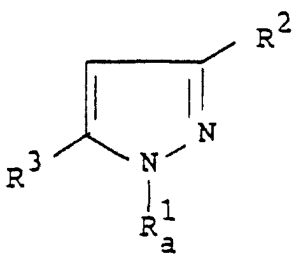

[Ie]

or its reactive derivative at the carboxy group or a salt thereof with a compound of the formula :

$$CH_2(COOR^4)_2 \qquad [V]$$

and then subjecting the resultant product to hydrolysis reaction to provide a compound of the formula :

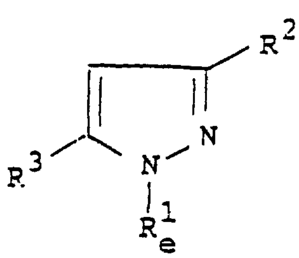

[Im]

in the above formulas,

$R^1_a$ is phenyl substituted with carboxy, $R^2$ and $R^3$ are each as defined in the present claim 1,

$R^1_e$ is phenyl substituted with acetyl, and

$R^4$ is $(C_1\text{-}C_6)$alkyl, or

f) reacting a compound of the formula :

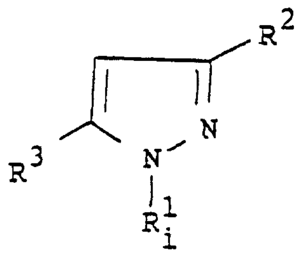

[Iq]

or its salt with an alkylating agent to provide a compound of the formula :

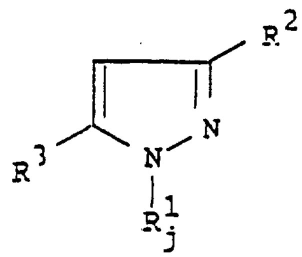

[Ir]

or its salt, in the above formulas,

$R_i^1$ is phenyl substituted with hydroxy,
$R_j^1$ is phenyl substituted with $(C_1\text{-}C_6)$alkoxy and
$R^2$ and $R^3$ are each as defined in the present claim 1.

**4.** A pharmaceutical composition comprising a compound of claim 1, as an active ingredient, in association with a pharmaceutically acceptable, substantially non-toxic carrier or excipient.

**5.** A compound of claim 1 for use as a medicament.

**6.** Use of a compound of claim 1 for the manufacture of a medicament for therapeutic treatment and/or prevention of inflammatory conditions, various pains, collagen diseases, autoimmune diseases or various immunity diseases in human beings or animals.

## Patentansprüche

**1.** Verbindung der Formel

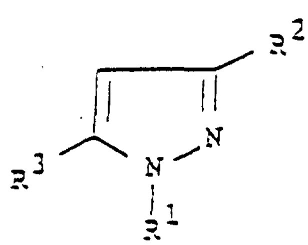

wobei

$R^1$ Phenyl ist, der mit Substituent(en) substituiert ist, welche ausgewählt werden aus der aus Cyano; $(C_1\text{-}C_6)$ Alkylendioxy; $(C_1\text{-}C_6)$Alkanoyl; und $(C_1\text{-}C_6)$Alkoxy bestehenden Gruppe;
$R^2$ Halogen; Halo$(C_1\text{-}C_6)$alkyl oder Cyano ist;

$R^3$ Phenyl ist, der substituiert ist mit ($C_1$-$C_6$)Alkylthio; ($C_1$-$C_6$)Alkylsulfinyl; oder ($C_1$-$C_6$)Alkylsulfonyl

vorausgesetzt, daß,
wenn

$R^1$ mit ($C_1$-$C_6$)Alkoxy substituiertes Phenyl ist, dann
$R^2$ Halogen oder Halo($C_1$-$C_6$)alkyl ist,

und pharmazeutisch verträgliche Salze davon.

**2.** Verbindung nach Anspruch 1, wobei

$R^1$ mit Cyano($C_1$-$C_6$)alkanoyl oder ($C_1$-$C_6$)Alkoxy substituiertes Phenyl ist und
$R^2$ Halogen oder Halo($C_1$-$C_6$)alkyl ist.

**3.** Verfahren zur Herstellung einer Verbindung nach Anspruch 1 und von pharmazeutisch verträglichen Salzen davon, welches umfaßt

a) Umsetzung einer Verbindung der Formel:

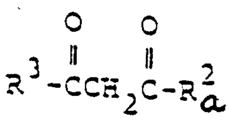

[II]

oder von deren Salz mit einer Verbindung der Formel:

$$R^1\text{-NH-NH}_2 \qquad [III]$$

oder mit deren Salz zur Herstellung einer Verbindung der Formel:

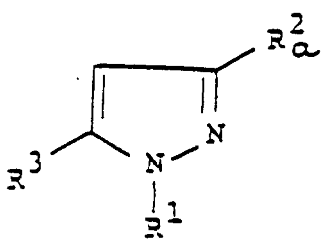

[Ia]

oder von deren Salz, wobei in den obigen Formeln $R^1$ und $R^3$ jeweils wie im Anspruch 1 definiert sind und $R^2_a$ Halo($C_1$-$C_6$)alkyl oder Cyano ist oder
b) Umsetzung einer Verbindung der Formel:

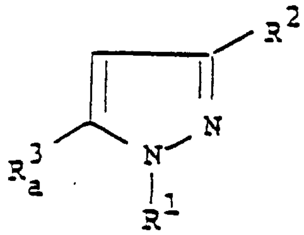

[Ib]

oder von deren Salz mit einem Oxidationsmittel zur Herstellung einer Verbindung der Formel:

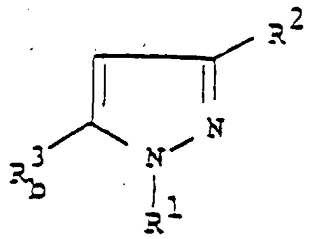

[Ic]

oder von deren Salz, wobei in den obigen Formeln

$R^1$ und $R^2$ jeweils wie im Anspruch 1 definiert sind,
$R^3{}_a$ mit ($C_1$-$C_6$)Alkylthio substituiertes Phenyl ist, und
$R^3{}_b$ mit ($C_1$-$C_6$)Alkylsulfinyl oder ($C_1$-$C_6$)Alkylsulfonyl substituiertes Phenyl ist, oder

c) Umsetzung einer Verbindung der Formel:

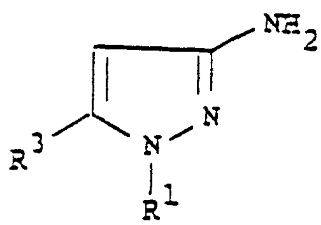

[IV]

oder von deren Salz mit einer Nitritverbindung, und dann Umsetzung des resultierenden Produkts mit Kupfer (I)-Halogenid zur Herstellung einer Verbindung der Formel:

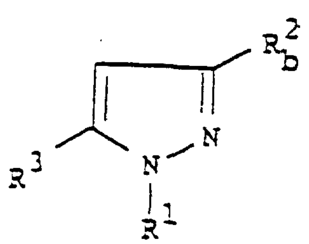

[Id]

oder von deren Salz, wobei in den obigen Formeln

$R^1$ und $R^3$ jeweils wie im Anspruch 1 definiert sind, und
$R^2_b$ Halogen ist, oder

d) Umsetzung einer Verbindung der Formel:

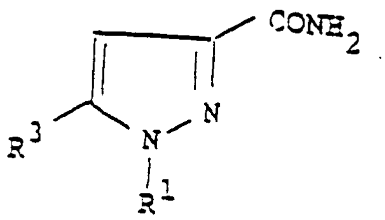

[Ik]

oder von deren Salz mit einem dehydratisierenden Mittel zur Bereitstellung einer Verbindung der Formel:

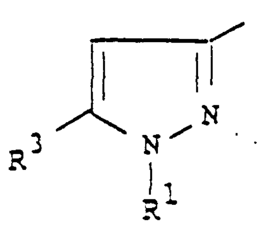

[Iℓ]

oder von deren Salz, wobei in den obigen Formeln

$R^1$ und $R^3$ jeweils wie in Anspruch 1 definiert sind, oder

e) Umsetzung einer Verbindung der Formel:

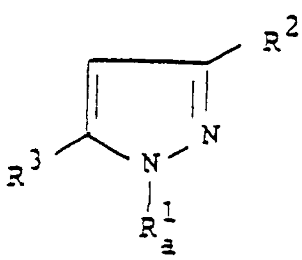

[Ie]

oder von deren reaktivem Derivat an der Carboxygruppe oder eines Salzes davon mit einer Verbindung der Formel:

$$CH_2(COOR^4)_2 \qquad [V]$$

und dann Durchführung einer Hydrolysereaktion mit dem resultierenden Produkt zur Bereitstellung einer Verbindung der Formel

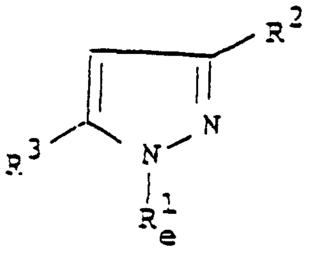

[Im]

wobei in den obigen Formeln

$R^1_a$ mit Carboxy substituiertes Phenyl ist, $R^2$ und $R^3$ jeweils wie in Anspruch 1 definiert sind,
$R^1_e$ mit Acetyl substituiertes Phenyl ist, und
$R^4$ ($C_1$-$C_6$)Alkyl ist, oder

f) Umsetzung einer Verbindung der Formel:

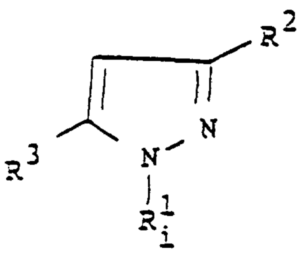

[Iq]

oder von deren Salz mit einem Alkylierungsmittel zur Bereitstellung einer Verbindung der Formel:

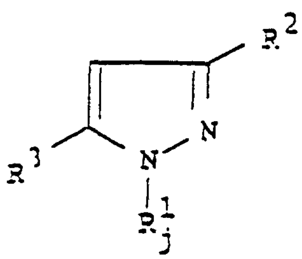

[Ir]

oder von deren Salz, wobei in den obigen Formeln

$R^1_i$ mit Hydroxy substituiertes Phenyl ist,
$R^1_j$ ($C_1$-$C_6$)Alkoxy substituiertes Phenyl ist, und
$R^2$ und $R^3$ jeweils wie in Anspruch 1 definiert sind.

**4.** Pharmazeutische Zusammensetzung, umfassend eine Verbindung nach Anspruch 1 als aktiven Bestandteil in Verbindung mit einem pharmazeutisch verträglichen, im wesentlichen nicht toxischen Träger oder Exzipienten.

**5.** Verbindung nach Anspruch 1 zur Verwendung als Medikament.

**6.** Verwendung einer Verbindung nach Anspruch 1 zur Herstellung eines Medikaments für die therapeutische Behandlung und/oder Vorbeugung von entzündlichen Zuständen, verschiedenen Schmerzen, Kollagenerkrankungen, Autoimmunerkrankungen oder verschiedenen Immunerkrankungen bei Menschen oder Tieren.

## Revendications

**1.** Composé de formule :

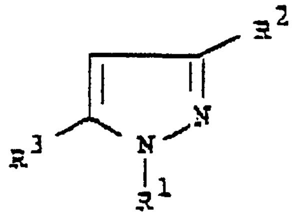

(I)

dans laquelle

$R^1$ représente un groupe phényle qui est substitué par un ou plusieurs substituant(s) choisi(s) dans le groupe constitué par des groupes cyano ; (alkylène en $C_1$-$C_6$)dioxy ; alcanoyle en $C_1$-$C_6$ ; et alcoxy en $C_1$-$C_6$ ;
$R^2$ représente un atome d'halogène ; un groupe halogéno(alkyle en $C_1$-$C_6$) ou cyano ;
$R^3$ représente un groupe phényle substitué par un groupe (alkyl en $C_1$-$C_6$)thio ; (alkyl en $C_1$-$C_6$)sulfinyle ; ou (alkyl en $C_1$-$C_6$)sulfonyle

à condition que
lorsque $R^1$ représente un groupe phényle substitué par un groupe alcoxy en $C_1$-$C_6$, $R^2$ représente un atome d'halogène ou un groupe halogéno(alkyle en $C_1$-$C_6$)
et sels pharmaceutiquement acceptables de celui-ci.

**2.** Composé selon la revendication 1, dans lequel

$R^1$ représente un groupe phényle substitué par un groupe cyano, alcanoyle en $C_1$-$C_6$ ou alcoxy en $C_1$-$C_6$, et
$R^2$ représente un atome d'halogène ou un groupe halogéno(alkyle en $C_1$-$C_6$).

**3.** Procédé de préparation d'un composé selon la revendication 1 et de sels pharmaceutiquement acceptables de celui-ci, qui comprend les étapes consistant à :

a) faire réagir un composé de formule :

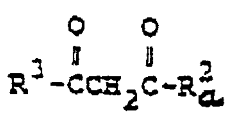

[II]

ou son sel avec un composé de formule :

$$R^1\text{-NH-NH}_2 \qquad [III]$$

ou son sel pour obtenir un composé de formule :

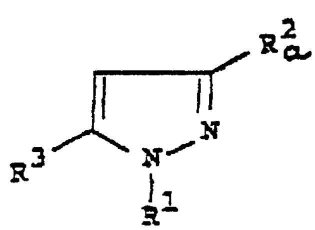

[Ia]

ou son sel, dans les formules ci-dessus,

$R^1$ et $R^3$ sont chacun tels que définis dans la présente revendication 1 et
$R^2_a$ représente un groupe halogéno-(alkyle en $C_1$-$C_6$) ou cyano, ou

b) faire réagir un composé de formule :

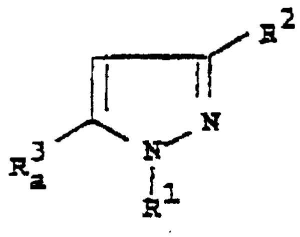

[Ib]

ou son sel avec un agent oxydant pour obtenir un composé de formule :

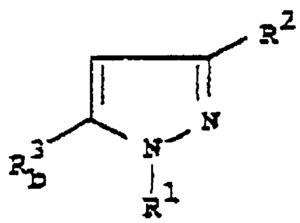

[Ic]

ou son sel, dans les formules ci-dessus,

$R^1$ et $R^2$ sont chacun tels que définis dans la présente revendication 1.
$R^3_a$ représente un groupe phényle substitué par un groupe (alkyl en $C_1$-$C_6$)thio, et
$R^3_b$ représente un groupe phényle substitué par un groupe (alkyl en $C_1$-$C_6$)sulfinyle ou (alkyl en $C_1$-$C_6$) sulfonyle, ou

c) faire réagir un composé de formule :

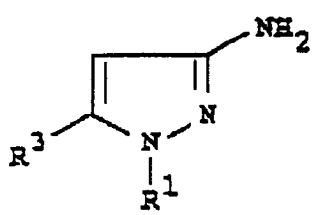

[IV]

ou son sel avec un composé de nitrite,
et faire réagir ensuite le produit résultant avec un halogénure cuivreux pour obtenir un composé de formule :

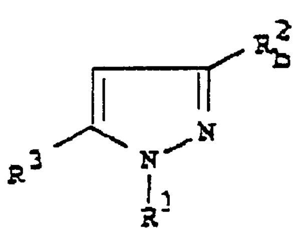

[Id]

ou son sel, dans les formules ci-dessus,

$R^1$ et $R^3$ sont chacun tels que définis dans la présente revendication 1, et $R^2_b$ représente un atome d'halogène, ou

d) faire réagir un composé de formule :

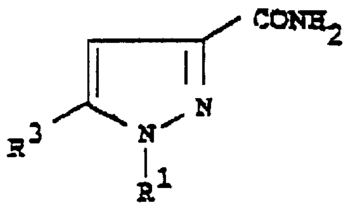

[Ik]

ou son sel avec un agent déshydratant pour obtenir un composé de formule :

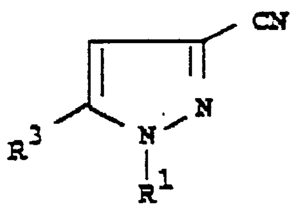

[Il]

ou son sel, dans les formules ci-dessus,

$R^1$ et $R^3$ sont chacun tels que définis dans la présente revendication 1, ou

e) faire réagir un composé de formule :

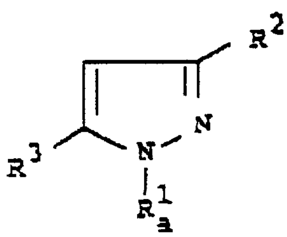

[Ie]

ou son dérivé réactif au niveau du groupe carboxy ou un sel de celui-ci avec un composé de formule :

$$CH_2(COOR^4)_2 \quad [V]$$

et soumettre ensuite le produit résultant à une réaction d'hydrolyse pour obtenir un composé de formule :

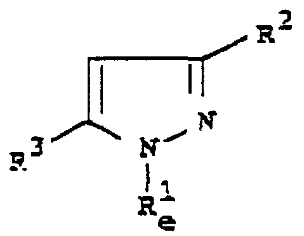

[Im]

dans les formules ci-dessus,

$R^1_a$ représente un groupe phényle substitué par un groupe carboxy, $R^2$ et $R^3$ sont chacun tels que définis dans la présente revendication 1,
$R^1_e$ représente un groupe phényle substitué par un groupe acétyle, et
$R^4$ représente un groupe (alkyle en $C_1$-$C_6$), ou

f) faire réagir un composé de formule :

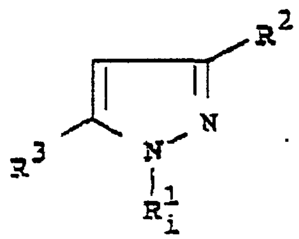

[Iq]

ou son sel avec un agent d'alkylation pour obtenir un composé de formule :

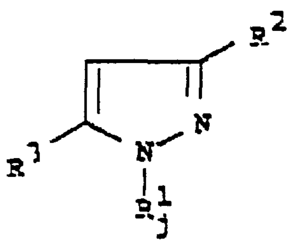

[Ir]

ou son sel, dans les formules ci-dessus,

$R^1_i$ représente un groupe phényle substitué par un groupe hydroxy,
$R^1_j$ représente un groupe phényle substitué par un groupe alcoxy en $C_1$-$C_6$, et
$R^2$ et $R^3$ sont chacun tels que définis dans la présente revendication 1.

**4.** Composition pharmaceutique comprenant un composé selon la revendication 1 comme constituant actif, en association avec un support ou un excipient pharmaceutiquement acceptable, essentiellement non toxique.

**5.** Composé selon la revendication 1, destiné à être utilisé comme médicament.

**6.** Utilisation d'un composé selon la revendication 1 pour la fabrication d'un médicament pour le traitement thérapeutique et/ou la prévention d'états inflammatoires, de diverses douleurs, de maladies du collagène, de maladies auto-immunes ou de diverses maladies du système immunitaire chez les êtres humains ou les animaux.